# EUROPÄISCHE PATENTSCHRIFT

(11) **EP 0 981 294 B1**
(45) Veröffentlichungstag und Bekanntmachung des Hinweises auf die Patenterteilung: **08.10.2003**
(21) Anmeldenummer: 98912188.4
(22) Anmeldetag: 16.04.1998
(51) Int. Cl.: A61B 7/02

(54) **STETHOSKOPKOPF**
STETHOSCOPE HEAD
TETE DE STETHOSCOPE

(30) Priorität: 13.05.1997 CH 111597
(43) Veröffentlichungstag der Anmeldung: 01.03.2000
(73) Patentinhaber: Granzotto, Artemio, 8005 Zürich (CH)
(72) Erfinder: Granzotto, Artemio, 8005 Zürich (CH)
(74) Vertreter: Patentanwälte Feldmann & Partner AG
(86) Internationale Anmeldenummer: CH9800142
(87) Internationale Veröffentlichungsnummer: WO98051221

(56) Entgegenhaltungen:
- EP-A- 0 094 183
- WO-A-89/09026
- DE-A- 4 029 122
- GB-A- 783 945

## Beschreibung

Die vorliegende Erfindung betrifft einen Stethoskopkopf nach Patentanspruch 1.

Obwohl die Einführung des Stethoskopes durch T. R. H. Laennec, dem Begründer der Auskultation, schon beinahe 180 Jahre zurückliegt stellt das mechanisch-akustische Stethoskop immer noch eines der wichtigsten und am häufigsten eingesetzten Diagnosegeräte in der täglichen Praxis der Mediziner dar. Das diagnostische Abhorchen der Organe, und vor allem des Herzens und der Lunge, auf Schallphänomene erschliesst dem erfahrenen Arzt eine Fülle an akustischer Information über das Befinden des Patienten.

Die ursprünglich eingesetzten offenen Hörrohre wurden durch die heute gebräuchlichen Stethoskope ersetzt, bestehend aus einem Kopf-oder Bruststück, einem Schlauchsystem mit bügelarmierten Ohroder Kopfhörern zur Schallübertragung in das Ohr. Zur Aufnahme des Körperschalles tragen die Kopfstücke glockenförmige Resonanzkörper, die offen oder von einer Membran abgedeckt sein können. Zwar eignen sich die offenen Resonanzkörper gut zum Erfassen von niederfrequenten Tönen, aber da die Haut des Patienten vollumfänglich dicht mit dem unteren Rand des Resonanzkörpers abschliessen muss, um eine befriedigende Funktion zu gewährleisten, müssen sie stark auf die Körperoberfläche des Patienten gepresst werden. Ist das System nicht geschlossen, so kann Schall nach aussen treten, Schallenergie geht verloren und die Effizienz des Stethoskopes nimmt ab. Das starke Anpressen des Resonanzkörpers kann einerseits schmerzhaft für den Patienten sein, und andererseits wird durch den starken Anpressdruck die Haut des Patienten aufgewölbt und dringt mehr oder weniger stark in den Glockenkörper ein. Dadurch wird der freie Resonanzraum verringert und dementsprechend muss der Resonanzkörper relativ hoch ausgebildet sein um ein vollständiges Ausfüllen durch die Haut zu verhindern.

Bei membrantragenden Resonanzkörpern ist das schaileitende System des Bruststückes üblicherweise geschlossen. Das Kopfstück muss daher während der Auskultation nicht oder nur schwach angepresst werden und die steife Membran liegt auf der Haut des Patienten auf. Da sich die Haut und die aufliegende Membran nicht oder nur schwach aufwölben, kann der Resonanzkörper wesentlich flacher ausgebildet werden. Die Steifigkeit der Membran dämpft allerdings niederfrequente Töne.

Um die Vorteile beider Systeme miteinander zu verbinden, wurden Zwei- oder sogar Mehr-weg-Stethoskope entwickelt, die einen offenen und einen geschlossenen Resonanztrichter in einem Kopfstück oder sogar mehrere Kopfstücke für verschieden Frequenzbereiche umfassen.

Um die Vorteile der membrantragenden Systeme nutzen und gleichzeitig niedrigen Frequenzen erfassen zu können wurden verschiedene Stethoskopköpfe entwickelt.

Ein Zweiwegstethoskopkopf mit einer schwimmend gelagerten Membran ist in der EP-A-0 119 870 beschrieben. Die Membran ist lose zwischen der Unterseite des Resonanzkörpers und dem unteren umlaufenden Rand eines am Resonanzkörper befestigten Rückhalteringes gelagert. Die Membran ist dadurch um eine Strecke, die etwas geringer als ihre Höhe ist, auf- und abbeweglich. Durch Variation im Anpressdruck sollen Membran und Resonanzkörper wahlweise ein offenes oder geschlossenes akustisches System bilden und dementsprechend die Auskultation von nieder- oder hochfrequenten Körpergeräuschen ermöglichen.

In der US-A-4 440 258 ist ein Stethoskopkopfstück beschrieben, dessen Membran in vertikaler Richtung zwischen zwei Relativpositionen in Bezug auf den Resonanzkörper bewegt werden kann, wobei die akustische Steifheit der Membran verändert wird und das Stethoskop auf verschiedene Frequenzhöhen abgestimmt werden kann.

In der US-A-4 995 473 ist ein Adapter aus Elastomermaterial für einen Stethoskopkopf beschrieben, der den basalen Bereich des Stethoskopkopfes umschliesst. Der hohlzylinderförmige Adapter verjüngt sich zur, dem Patienten zugewandten Seite hin. Wird der mit dem Adapter versehene Stethoskopkopf auf die Körperoberfläche des Patienten aufgelegt, so wird der Luftraum zwischen Körperoberfläche und Membran schalldicht gegen die Umgebung abgedichtet, ohne dass die Membran mit der Körperoberfläche in Berührung kommt.

Aus der EP-A-0 500 279 ist ein ergonometrisches Bruststück eines Stethoskopes bekannt. Ein erhöhtes Mittelstück mit zwei seitlichen Griffmulden ist dem Resonanzkörper aufgesetzt ist, um das Greifen und Halten des Stethoskopkopfes zu erleichtern und gleichzeitig die Bedienung von Regelelementen zu ermoglichen, die zur Kontrolle der Lautstärke am Mittelstück angebracht sind.

Der nächste Stand der Technik nach WO-A-8909026 ist im Oberbegriff von Patentanspruch 1 gewärdigt.

Beim Erfassen und beim Auswerten der akustischen Signale werden hohe Ansprüche an das Stethoskop und auch an den untersuchenden Mediziner gestellt. Um die akustische Information optimal nutzen zu können, und dadurch Fehldiagnosen zu vermeiden, ist also einerseits viel Erfahrung auf Seiten des Arztes nötig, andererseits muss das vom Stethoskop gelieferte Signal von hoher Qualität, reproduzierbar und von möglichen äusseren Störsignalen möglichst frei sein.

Zur Erleichterung und Unterstützung der Diagnose wird seit langem versucht die vom Körper ausgehenden Geräusche durch Mikrophone und Verstärker akustisch zu verstärken. Der Einsatz von Mikrocomputern und massgeschneiderter Diagnose-Software in der Phonokardiographie ist ein weiterer Schritt zur Erleichterung der Interpretation der Schallphänomene. Die zunehmende Miniaturisierung von Elekronik- und Computerbauteilen hat dazu geführt, dass solche Systeme inzwischen in handliche Geräte, im Idealfall in einem etwas vergrössierten Stethoskopkopf, integriert erhältlich sind.

Ob nun herkömmlich der Direktschall oder computerunterstutzt ein in elektrische Spannungsänderungen transformiertes Signal ausgewertet wird, die Qualität der erhaltenen Information hängt immer entscheidend von der Qualität des primär an der Körperoberfläche aufgenommenen akustischen Signals ab. Dieses Primärsignal wird durch verschiedene Faktoren negativ beeinflusst. Geräusche aus der Umgebung werden vom Resonanzkörper aufgenommen und weitergeleitet. Geräusche, die als Substratschall durch das Halten des Resonanzkörpers, oder das Auflegen des Resonazkörpers oder von Teilen, die in ungedämmtem Kontakt mit dem Resonanzkörper stehen, auf die Körperoberfläche des Patienten entstehen, erzeugen störende Geräusche und Interferenzen. Zusätzlich stellt sich das Problem, dass durch Unterschiede im Anpressdruck des Stethoskopkopfes an die Körperoberfläche die Reproduzierbarkeit der abgehörten Signale nicht gewährleistet ist.
Insbesondere die vom Arzt selber stammenden Signale werden über dessen Hand an den Stethoskopkopf übertragen und führen zu störenden übergelagerten Fremdsignalen.

Die vorliegende Erfindung stellt sich daher die Aufgabe, einen Stehtoskopkopf zur Verfügung zu stellen, die die genannten Nachteile nicht aufweisen.

Diese Aufgabe wird durch eine Vorrichtung gemäss der kennzeichnenden Merkmale des Anspruches 1 gelöst.
Weitere Ausführungsvarianten ergeben sich aus den abhängigen Ansprüchen.

In den Zeichnungen ist ein Ausführungsbeispiel des Erfindungsgegenstandes dargestellt und in der nachfolgenden Beschreibung erläutere. Es zeigt:
- Figur 1: einen Querschnitt durch den Stethoskopkopf entlang der Medianebene; und
- Figur 2: einen Querschnitt durch den Stethoskopkopf entlang der Frontalebene.
- Figur 3: eine Detailvergrösserung eines Querschnittes entlang der Medianebene durch den peripheren Bereich einer weiteren Ausführungsform des Stethoskopkopfes.

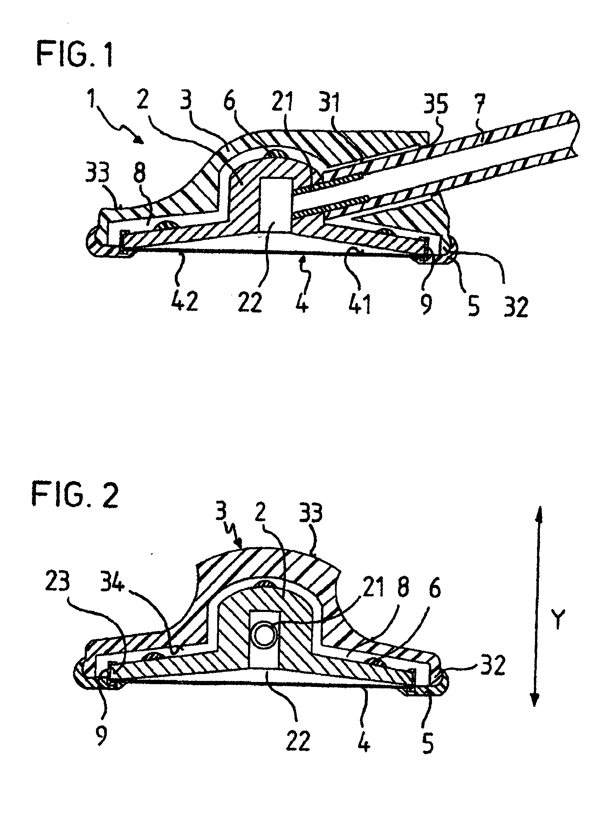

Der in den Figuren 1 und 2 dargestellte Stethoskopkopf 1 besitzt einen glockenförmigen Resonanzkörper 2 aus einem geeigneten Metall, wie Aluminium, Stahl, Eisen oder Messing. Resonanzkörper aus Kunststoffen sind auch bekannt, allerdings infolge ihrer schlechteren akustischen Eigenschaften nicht sehr vorteilhaft. Seitlich am Resonanzkörper 2 angeordnet ist ein Anschlusstutzen 21, auf den ein Schlauch 7 aus Kunststoff oder Gummi aufgesteckt ist. Der Resonanzkörper 2 besitzt einen peripheren, ununterbrochenen Kranz 23, der an der Basaiseite, die im Einsatz der Körperoberfläche des Patienten zugewandt ist, eine Basalfläche 24 aufweist. Der periphere Bereich einer flachen scheibenförmigen Membran 4 wird zwischen dieser Basalfläche 24 und einer Klemmfläche 91 eines Halteringes 9 form- und kraftschlüssig klemmend gehalten. Da die Basaifläche 24, Ober- 41 und Unterseite 42 der Membran 4 und die Klemmfläche 91 in annähernd parallelen Ebenen liegen, und da die Membran über 360° klemmend gehalten ist, wirken rundum aleichmässige Klemmkräfte auf die Membran. Ein Vertikalzylinder 92 des Halteringes 9 weist eine nach innen vorspringende umlaufende basale oder proximale Halterippe 94 auf. Diese trägt die Klemmfläche 91. Der Haltering 9 ist mit dem peripheren Kranz 23 des Glockenkörpers 2 verschraubt, oder ist aussen über den Kranz 23 gestülpt, und mittels einer zusätzlichen distalen Halterippe 95 klemmend an ihm gehalten.

Der Resonanzkörper 2 und die Membran 4 schliessen einen Resonanzraum 22 ein. Ueber den Stutzen 21 steht die Luft im Resonanzraum 22 in kommunizierender Verbindung mit der Luftsaule des Schlauches 7. Die bei einer Untersuchung der Körperoberfläche des Patienten abgewandte oder distale Seite des Resonanzkörpers 2 ist vollständig von einer schallisolierenden Kapsel 3 aus Kunststoff umgeben. Die Kapsel 3 ist ergonomisch geformt, so dass sie dem Arzt möglichst gut in der Hand liegt. Damit der Stethoskopkopf auch mit feuchten Händen möglichst griffsicher gehalten werden kann, ist die äussere Oberfläche 33 der Kapsel 3 strukturiert, oder die Kapsel 3 ist mit einer zusätzlichen Anti-Rutsch-Beschichtung versehen. Die Kapsel 3 weist eine seitliche Durchlassöffnung 31 auf, durch die der am Resonanzkörper 2 befestigte Schlauch 7 hindurchgeführt ist. Eine proximale Seite 34 der Kapsel 3 oder die Seite, die dem Resonanzkörper 2 zugewandt ist, ist derart geformt, dass überall zwischen Kapsel 3 und Resonanzkörper 2 ein durchgehender, mindestens einige Millimeter breiter gas- oder luftgefüllter Spalt 8 besteht. Die Kapsel 3 berührt an keiner Stelle den Resonanzkörper 2 solange von aussen kein starker Druck auf sie ausgeübt wird. Der Luftspalt 8 setzt sich auch entlang des Schlauches 7 bis nahe zu einer Austrittsstelle 35 des Schlauches 7 aus der Kapsel 3 fort. Hier wird der Schlauch 7 auf einer Länge von mehreren Millimetern dicht vom Dämmmaterial der Kapsel 3 umschlossen, so dass der Luftspalt 8 gasdicht oder wenigstens annähernd gasdicht gegen die umgebende Atmosphäre abgeschlossen ist. Die Abdichtung der Kapsel 3 gegen den Schlauch 7 kann alternativ auch mittels einer separaten Dichtung aus Dämmaterial erfolgen.

Die aus schalldämmendem Material hergestellte Kapsel 3 wirkt teilweise wie ein zweiter Resonanzkörper, der die Gesamtresonanz so verstärkt, dass die auskultierten Töne an Klarheit und Lautstärke gewinnen.

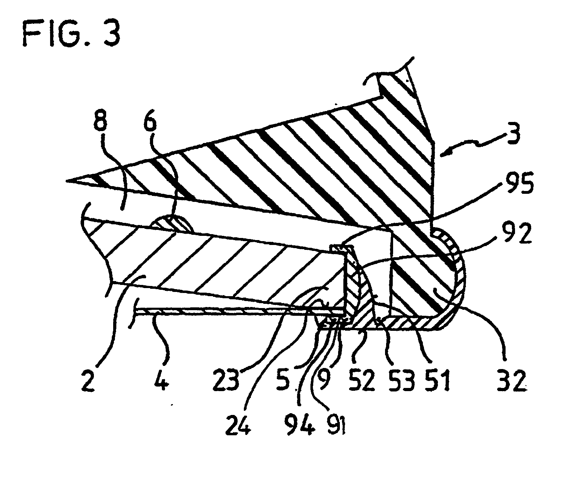

Die Wandung der Kapsel 3 ist im Bereich ihres proximalen Randes 32 nach aussen hin wulstförmig verdickt. An diesem Wulst 32 und am Haltering 9 greift eine umlaufende dichtende Aufhängung 5 an, die den Luftspalt 8 zwischen Kapsel 3 und dem am Resonanzkörper 2 befestigten Haltering 9 überbrückt. Der Resonanzkörper 2, die Membran 4 und der Halteringe 9 bilden ein festes System. Sie sind daher gemeinsam über die Aufhängung 5 beweglich mit der Kapsel 3 verbunden. Das Material, aus dem die Aufhängung 5 gefertigt ist, ist elastisch genug um eine eingeschränkte Relativbeweglichkeit des Resonanzkörpers 2 zur Kapsel 3 zu ermöglichen. Die Aufhängung 5 erstreckt sich über die gesamte Breite der basalen Halterippe 94 des Halteringes 9 und liegt einer Basalseite 42 der Membran 4 voliumfänglich auf einer Breite von etwa 0.5 mm dicht an. Die Aufhängung 5 gewährleistet dadurch die Schalldämmung zwischen der Körperoberfläche des Patienten und dem Resonanzkörper 2 auf zwei Wegen. Es wird der Substratschall zwischen Körper und Haltering 9 und dem damit verbundenen Resonanzkörper 2 ebenso gedämpft, wie der Luftschall zwischen der Körperoberfläche und dem Luftspalt 8, der den Resonanzkörper 2 umgibt. Die Aufhängung 5 kann im Bereich, der den Luftspalt zwischen Kapsel 3 und Resonanzkörper 2 überbrückt, auf verschiedene Arten gestaltet sein. In einer vorteilhaften Ausgestaltung der vorliegenden Erfindung sind eine distale Oberfläche 51 und eine proximale Oberfläche 52 der Aufhängung 5 im Bereich, der den Luftspalt zwischen Kapsel 3 und Resonanzkörper 2 überbrückt, annähernd parallel zueinander, wobei die Aufhängung in diesem Bereich zwischen 0.1 und 1.0 mm, vorzugsweise 0.3 mm dick ist. Die Aufhängung 5 kann in diesem Bereich auch dicker sein, und wie in Figur 3 dargestellt eine plane Proximalfläche 52 und eine geschwungene Distalfläche 51 aufweisen. Ist die Aufhängung in diesem Bereich dicker als 1.0 mm, so wird vorteilhafterweise an der Distalseite 51 eine umlaufende Vertiefung oder Rille 53 angebracht, die U- oder V-förmig gestaltet ist, und die Dicke der Aufhängung 5 an ihrer dünnsten Stelle auf 0.1 bis 1.0 mm, vorteilhafterweise 0.3 mm, verringert.

Der Resonanzkörper 2, und damit auch die mit ihm verbundene Membran 4, können in Relation zur Kapsel 3 entlang der Achse Y um einige Millimeter verschoben sowie um einige Grad verkippt werden. Die Auslenkung ist einerseits durch die Aufhängung 5 und andererseits durch die lichte Weite des Luftspaltes 8 begrenzt. Um auch bei starker Auslenkung eine direkte Berührung des Resonanzkörpers 2 mit der Innenseite der Kapsel 3 zu verhindern, sind vibrationshemmende Isolationsnoppen 6 aus weichem Elastomermaterial auf der Distalseite des Resonanzkörpers 2 angebracht. Die Höhe der Noppen 6 ist geringer'als die lichte Weite des Luftspaltes 8 im Ruhezustand. Wird also die Kapsel 3 mit höherem Druck auf die Körperoberfläche gepresst, so wird durch den Gegendruck dieser auf das membrantragende die Isolationsnoppen 6 gegen die Innenseite der Kapsel 3 gedrückt. Die weich-elastischen Noppen 6 können einen Teil der einwirkenden Energie durch Verformen absorbieren, leiten jedoch mit zunehmendem Druck mehr Druck auf den Resonanzkörper 2 weiter. Falls der untersuchende Arzt dies wünscht, kann er also durch stärkeren Druck auf die Kapsel 3 die elastische Aufhängung des Stethoskopkopfes ausser Kraft setzen und die Membran 4 mit beliebiger Stärke anpressen. Es ist jedoch in jedem Fall die Schallisolierung zwischen Kapsel 3 und Resonanzkörper 2 gewährleistet.

Versuche des Anmelders haben gezeigt, dass die qualitativ besten Auskultationsergebnisse mit hochwertige Stethoskopen bei einem gleichmässigen niedrigen Anpress- oder Auflagedruck erhalten werden. Der Druck mit dem die Membran 4 auf der Körperoberfläche aufliegt, wird idealerweise durch das Eigengewicht des Resonanzkörpers 2, welches normalerweise im Bereich von 60-70 Gramm liegt, bestimmt. Solche niedrige Auflagedrücke können jedoch von Hand nur schwierig erzeugt und kontrolliert werden. Bei dem vorliegenden Stethoskopkopf 1 kann der untersuchende Arzt den gekapselten Stethoskopkopf 1 unterschiedlich stark auf die Körperoberfläche des Patienten drücken, ohne dass sich der Druck auf die Membran 4 dadurch wesentlich ändert, da die elastische Lagerung 5 ein Zurückweichen des membrantragenden Systems zulässt. Die gesamte Einheit aus Membran 4, Haltering 9 und Resonanzkörper 2 wird also durch Druck auf die Membran 4 und die unter dem Haltering 9 liegenden Bereiche der Aufhängung 5 zur Kapsel hin verschoben. Stärkerer Druck auf den Stethoskopkopf hat bei einer herkömmlichen, starr am Stethoskopkopf gelagerten Membran eine mehr oder weniger ungleichmässige Wölbung und ungleichmässig verteilte Druckkräfte über den gesamten Bereich der Membran zur Folge. Da die erfindungsgemässe Aufhängung 5 aber in beschränktem Masse ein vertikales Ausweichen und ein laterales Verkippen der Membran 3 und des gesamten membrantragenden Systems in Relation zu der vom untersuchenden Arzt gehaltenen Kapsel 3 zulässt, kann die Membran 4 unter Wahrung ihrer Form der Körperoberfläche aufliegen, wobei eine relativ homogene Druckverteilung über die gesamte Fläche der Membran 4 erreicht wird. Dies verbessert die akustischen Eigenschaften des Stethoskopkopfes 1 ganz entscheidend.

Zusätzlich stellt die Aufhängung 5, die dicht mit der Kapsel 3 und auch dicht mit der Basalseite 42 der Membran 4 abschliesst eine wirksame Barriere für Flüssigkeiten und Feststoffe dar. Sie verhindert deren Eindringen in den Luftspalt 8 und damit die Kontamination und Verschmutzung innenliegender Bereiche der Kapsel 3 und des Resonanzkörpers 2. Durch die erfindungsgemässe Kapselung weist der Stethoskopkopf 1 eine nahezu nahtlos geschlossene Oberfläche auf, die seine Reinigung und Desinfektion sehr einfach macht. Da das Stethoskop im hygienisch sensiblen Bereich zum Einsatz kommt, und das Risiko pathogene Keine zu übertragen minimiert werden muss, ist dies von entscheidendem Vorteil. Für das Material aus dem die Aufhängung 5 geformt ist, bedeutet dies, dass es auch aggressiven und abrasiven Desinfektionsmitteln und -methoden gut standhalten muss.

In einer weiteren vorteilhaften Ausgestaltungsform der Erfindung sind die Aufhängung 5 und der Haltering 9 einstückig geformt. Dies ist besonders kostengünstig. Auch die Andruckkraft der Membran 4 am Resonanzkörper 2 kann so möglichst gering gehalten werden.

Wie aus Figur 1 ersichtlich ist, kommt während der Auskultation kein aus Metall gefertigtes Teil des Stethoskopkopfes 1 mit der Körperoberfläche des Patienten in direkte Berührung. Nur die Membran 4 und die Aufhängung 5 stehen mit dem Patientenkorper in Kontakt. Da beide aus Materialien mit einer niedrigen Wärmeleitfähigkeit gefertigt sind, kommt es beim Auflegen des Stethoskopkopfes 1 auf die Körperoberfläche nicht zu einem für den Patienten unangenehmen Kontakt mit kaltem Metall. dieser "Kälteschock" kann gerade bei Säuglingen und Kleinkindern heftige Reaktionen hervorrufen und deren Untersuchung äusserst schwierig und langwierig gestalten.

Ein allfälliges Vorwärmen des Kopfstückes 1 vor der Untersuchung ist bei der vorliegenden Erfindung nicht mehr nötig.

Auf ein Gerät zum Temperieren des Stethoskopes, wie es zum Beispiel in EP-A- 0 179 971 beschrieben ist, kann ebenfalls verzichtet werden.

Da es während der Auskultation keinen direkten Kontakt zwischen dem metallenen Resonanzkörper 2 des Stethoskopkopfes 1 und der Körperoberfläche des Patienten gibt, ist es nicht nötig bei der Wahl der Metallegierungen auf deren allergene Eigenschaften zu achten. Die Auswahl kann allein von den akustischen Qualitäten bestimmt sein. Für die Membran 4 und die aus Kunststoff gefertigte Kapsel 3 und Aufhängung 5 werden vorteilhafterweise hypoallergene Materialien gewählt.

## Patentansprüche

1. Stethoskopkopf (1) für ein Einwegstethoskop mit einem trichter- oder glockenförmigen Resonanzkörper (2), der einen peripheren ununterbrochen umlaufenden Kranz (23) besitzt, der eine Basalfläche (24) aufweist, wobei zwischen der Basalfläche (24) und einer Klemmfläche (91) eines Halteringes (9) der periphere Bereich einer planen kreisscheibenförmige Membran (4) form- und kraftschlüssig klemmend gehalten ist, so dass von der Membran (4) und dem Resonanzkörper (2) ein Resonanzraum (22) umschlossen wird, der mit der Luftsäule im Lumen eines Schlauches (7) in kommunizierender Verbindung steht, und wobei eine Kapsel (3) aus schallisolierendem Material den distalen Bereich des Resonanzkörpers (2) vollständig umgibt, wobei eine Durchlassöffnung (31) für den Schlauch (7) ausgespart ist und eine Aufhängung (5) eine Verbindung zwischen der Kapsel (3) und dem am peripheren Kranz (23) des Resonanzkörpers (2) befestigten Haltering (9) bildet **dadurch gekennzeichnet, dass** die Kapsel (3) den distalen Bereich des Resonanzkörpers (2) berührungsfrei umgibt, und die Aufhängung (5) aus Elastomermaterial besteht und eine bewegliche Verbindung zwischen der Kapsel (3) und Haltering (9) bildet.

2. Stethoskopkopf (1) nach Anspruch 1, **dadurch gekennzeichnet, dass** die Aufhängung (5) einerseits an einem peripheren Wulst (32) der Kapsel (3) und andererseits am Haltering (9) befestigt ist, wobei die Aufhängung (5) sich über die gesamte Breite einer basalen umlaufenden Halterippe (94) des Halteringes (9) erstreckt und einer Basalseite (42) der Membran (4) vollumfänglich formschlüssig anliegt.

3. Stethoskopkopf (1) nach Anspruch 2, **dadurch gekennzeichnet, dass** zwischen der Kapsel (3) und dem Resonanzkörper (2) ein durchgehender Luftspalt (8) besteht, der im basalen Bereich von der Aufhängung (5) zwischen Kapsel (3) und dem am Resonanzkörpers (2) angebrachten Haltering (9) abgeschlossen ist.

4. Stethoskopkopf (1) nach Anspruch 1, **dadurch gekennzeichnet, dass** an der Distalseite des Resonanzkörpers (2) mindestens eine Isolationsnoppe (6) aus Elastomermaterial angebracht sind, deren Höhe geringer als die lichte Weite des Luftspaltes (8) ist.

5. Stethoskopkopf (1) nach Anspruch 1, **dadurch gekennzeichnet, dass** die Aufhängung (5) und der Haltering (9) einstückig geformt sind.

6. Stethoskopkopf (1) nach Anspruch 1, **dadurch gekennzeichnet, dass** die Kapselung (3) ergonomisch geformt ist.

7. Stethoskopkopf (1) nach Anspruch 1, **dadurch gekennzeichnet, dass** die Kapselung (3) aus schalldämpfendem Elastomermaterial oder Schaumkunststoffmaterial geformt ist.

8. Stethoskopkopf (1) nach Anspruch 2 oder 5, **dadurch gekennzeichnet, dass** die Aufhängung (5) im Bereich, der den Luftspalt (8) überspannt, zwischen 0.1 und 1.0 mm dick ist.

9. Stethoskopkopf (1) nach Anspruch 2 oder 5, **dadurch gekennzeichnet, dass** die Aufhängung (5) im Bereich, der den Luftspalt (8) überspannt, eine umlaufende Vertiefung (53) aufweist, so dass die Aufhängung an der dünnsten Stelle in diesem Bereich zwischen 0.1 und 1.0 mm dick ist.

10. Stethoskopkopf (1) nach Anspruch 1, **dadurch gekennzeichnet, dass** die Membran (4) aus Plastik, Fieberglas, Epoxyharz oder dünnem Metallblech mit einer glatten basalen Oberfläche (42) besteht.

11. Stethoskopkopf (1) nach Anspruch 1, **dadurch gekennzeichnet, dass** Membran (4), Aufhängung (5) und Kapsel (3) aus hypoallergenen Materialien gefertigt sind.

## Claims

1. A stethoscope head (1) for a disposable stethoscope with a funnel-like or bell-like resonance body (2) which has a peripheral uninterrupted circumferential rim (23) which has a basal surface (24), wherein between the basal surface (24) and a clamping surface (91) of a retaining ring (9) the peripheral region of a planar circular-disk-shaped membrane is clampingly held with a positive and non-positive fit, so that a resonance space (22) is enclosed by the membrane (4) and the resonance body (2), said space being in communicating connection with the air column in the lumen of a flexible tubing (7), and wherein a capsule (3) of sound-insulating material completely surrounds the distal region of the resonance body (2), wherein a passage opening (31) for the flexible tubing (7) is relieved and a support (5) forms a connection between the capsule (3) and the retaining ring (9) fastened on the peripheral rim (23) of the resonance body (2), **characterised in that** the capsule (3) surrounds the distal region of the resonance body (2) without contact, and the support (5) consists of elastomeric material and forms a movable connection between the capsule (3) and retaining ring (9).

2. A stethoscope head (1) according to claim 1, **characterised in that** the support (5) is fastened on the one hand on a peripheral bead (32) of the capsule (3) and on the other hand on the retaining ring (9), wherein the support (5) extends over the whole width of a basal circumferential retaining rib (94) of the retaining ring (9) and bears with a positive fit on a basal side (42) of the membrane (4) over the whole circumference.

3. A stethoscope head (1) according to claim 2, **characterised in that** between the capsule (3) and the resonance body (2) there exists a continuous air gap (8) which in the basal region is closed off by the support (5) between the capsule (3) and the retaining ring (9) attached on the resonance body (2).

4. A stethoscope head (1) according to claim 1, **characterised in that** on the distal side of the resonance body (2) there is attached at least one insulation nap (6) of elastomer material whose height is smaller than the clear width of the air gap (8).

5. A stethoscope head (1) according to claim 1, **characterised in that** the support (5) and the retaining ring (9) are formed as one piece.

6. A stethoscope head (1) according to claim 1, **characterised in that** the capsule (3) is formed ergonomically.

7. A stethoscope head (1) according to claim 1, **characterised in that** the capsule (3) is formed of sound-damping elastomer material or foam plastic material.

8. A stethoscope head (1) according to claim 2 or 5, **characterised in that** the support (5) in the region which spans the air gap (8) is between 0.1 and 1.0 mm thick.

9. A stethoscope head (1) according to claim 2 or 5, **characterised in that** the support (5) in the region which spans the air gap (8) comprises a circumferential recess (53) so that the support at the thinnest location in this region is between 0.1 and 1.0 mm thick.

10. A stethoscope head (1) according to claim 1, **characterised in that** the membrane (4) consist of plastic, fibreglass, epoxy resin or thin sheet metal, with a smooth basal surface (42).

11. A stethoscope head (1) according to claim 1, **characterised in that** the membrane (4), support (5) and capsule (3) are manufactured of hypoallergenic materials.

## Revendications

1. Tête de stéthoscope (1) pour un stéthoscope à usage unique avec un corps de résonance (2) en forme d'entonnoir ou de cloche possédant une couronne périphérique ininterrompue (23) et présentant une surface basale (24), dans laquelle la zone périphérique d'une membrane plane en forme de disque (4) est retenue par ajustement et par force entre la surface basale (24) et la surface de serrage (91) d'un anneau de blocage (9), de sorte que la membrane (4) et le corps de résonance (2) enclosent une chambre de résonance (22) qui est en communication avec la colonne d'air contenue dans la lumière d'un tuyau (7), et dans laquelle une capsule (3) en matériau insonorisant entoure complètement la zone distale du corps de résonance (2), une ouverture de passage (31) étant ménagée pour le tuyau (7) et une attache (5) formant une liaison entre la capsule (3) et l'anneau de blocage (9) fixé sur la couronne périphérique (23) du corps de résonance (2), **caractérisée par le fait que** la capsule (3) entoure sans contact la zone distale du corps de résonance (2) et que l'attache (5) est en matériau élastomère et forme une liaison mobile entre la capsule (3) et l'anneau de blocage (9).

2. Tête de stéthoscope (1) selon la revendication 1, **caractérisée par le fait que** l'attache (5) est fixée d'un côté à un bourrelet périphérique (32) de la capsule (3) et de l'autre côté à l'anneau de blocage (9), l'attache (5) s'étendant sur toute la largeur d'une nervure de retenue périphérique basale (94) de l'anneau de blocage (9) et repose par ajustement géométrique sur toute la circonférence d'une face basale (42) de la membrane (4).

3. Tête de stéthoscope (1) selon la revendication 2, **caractérisée par le fait qu'**il existe entre la capsule (3) et le corps de résonance (2) une fente d'air traversante (8) enclose dans la zone basale de l'attache (5) entre la capsule (3) et l'anneau de blocage (9) monté sur le corps de résonance (2).

4. Tête de stéthoscope (1) selon la revendication 1, **caractérisée par le fait qu'**il est prévu sur la face distale du corps de résonance (2) au moins un bouton d'isolation (6) en matériau élastomère dont la hauteur est plus faible que la largeur intérieure de la fente d'air (8).

5. Tête de stéthoscope (1) selon la revendication 1, **caractérisée par le fait que** l'attache (5) et l'anneau de blocage (9) sont formés d'une seule pièce.

6. Tête de stéthoscope (1) selon la revendication 1, **caractérisée par le fait que** la capsule (3) est de forme ergonomique.

7. Tête de stéthoscope (1) selon la revendication 1, **caractérisée par le fait que** la capsule (3) est en matériau élastomère ou en matériau plastique cellulaire ou moussé.

8. Tête de stéthoscope (1) selon la revendication 2 ou 5, **caractérisée par le fait que** l'attache (5) a une épaisseur comprise entre 0,1 et 1,0 mm dans la zone recouvrant la fente d'air (8).

9. Tête de stéthoscope (1) selon la revendication 2 ou 5, **caractérisée par le fait que** l'attache (5) présente un évidement périphérique (53) dans la zone recouvrant la fente d'air (8), de sorte que l'attache (5) a une épaisseur comprise entre 0,1 et 1,0 mm à l'endroit le plus fin de cette zone.

10. Tête de stéthoscope (1) selon la revendication 1, **caractérisée par le fait que** la membrane (4) est en plastique, fibre de verre, résine époxyde ou fine tôle métallique avec une surface basale lisse (42).

11. Tête de stéthoscope (1) selon la revendication 1, **caractérisée par le fait que** la membrane (4), l'attache (5) et la capsule (3) sont réalisées en matériaux hypoallergéniques.
